# EUROPEAN PATENT SPECIFICATION

(11) **EP 3 203 215 B1**
(45) Date of publication and mention of the grant of the patent: **14.08.2019**
(21) Application number: 16154703.9
(22) Date of filing: 08.02.2016
(51) Int. Cl.: G01N 21/64, G01N 21/65, G01J 3/02, G01J 3/28, G01J 3/44, G01N 21/31, G01N 21/35, G01N 21/49, G01N 21/55, G02B 21/00, A61B 5/00

(54) **OPTICAL IMAGING SPECTROSCOPY OF LARGE-AREA SAMPLES**
OPTISCHE BILDGEBENDE SPEKTROSKOPIE VON GROSSFLÄCHIGEN PROBEN
OPTIQUE SPECTROSCOPIE D'IMAGERIE D'ÉCHANTILLONS À GRANDE SURFACE

(43) Date of publication of application: 09.08.2017
(73) Proprietor: Leibniz-Institut für Astrophysik Potsdam (AIP), 14482 Potsdam (DE)
(72) Inventor: SCHMÄLZLIN, Elmar, 14473 Potsdam (DE); MORALEJO, Benito, 12015 Berlin (DE); ROTH, Martin, 14547 Beelitz (DE)
(74) Representative: Müller & Schubert

(56) References cited:
- EP-A1- 0 717 296
- WO-A1-2009/020450
- WO-A1-2013/129755
- US-A1- 2006 052 709
- US-A1- 2010 265 498
- ANTHONY TSIKOURAS ET AL: "High-speed multifocal array scanning using refractive window tilting", BIOMEDICAL OPTICS EXPRESS, vol. 6, no. 10, 2 September 2015 (2015-09-02), page 3737, XP055302135, United States ISSN: 2156-7085, DOI: 10.1364/BOE.6.003737
- BRÜCKNER MICHAEL ET AL: "Fiber array based hyperspectral Raman imaging for chemical selective analysis of malaria-infected red blood cells", ANALYTICA CHIMICA ACTA, vol. 894, 29 August 2015 (2015-08-29), pages 76-84, XP029280954, ISSN: 0003-2670, DOI: 10.1016/J.ACA.2015.08.025
- ANTHONY TSIKOURAS ET AL: "Streak camera crosstalk reduction using a multiple delay optical fiber bundle", OPTICS LETTERS, OPTICAL SOCIETY OF AMERICA, US, vol. 37, no. 2, 15 January 2012 (2012-01-15), pages 250-252, XP001572698, ISSN: 0146-9592, DOI: 10.1364/OL.37.000250 [retrieved on 2012-01-13]
- ELMAR SCHMÄLZLIN ET AL: "Ultrafast imaging Raman spectroscopy of large-area samples without stepwise scanning", JOURNAL OF SENSORS AND SENSOR SYSTEMS, vol. 5, no. 2, 13 July 2016 (2016-07-13), pages 261-271, XP055302039, DOI: 10.5194/jsss-5-261-2016

## Description

The present invention relates to imaging spectroscopy of large-area samples, a device to perform imaging spectroscopy and the use of the device for imaging large-area samples, preferably of organic and inorganic material.

When light impinges on a chemical sample, usually fluorescence arises from the sample. If the excitation light is monochromatic and shows sufficient intensity, the scattered light shows Raman lines, i.e. color shifts that are related to the vibration states of the impinged molecules and the crystal structures. The Raman lines form a spectral fingerprint that allows a non-contacting and label-free identification of chemical structures. Raman lines are extremely faint (only a fraction of 1 × 10⁻⁷ of the scattered light is Raman scattering). However, based on considerable progress in laser and detector technologies, Raman spectroscopy have become more and more common, particularly, in the fields of medical diagnostics and life sciences. In contrast to the traditional infrared absorption spectroscopy, Raman spectroscopy is not impeded by the infrared light absorption of water. Raman lines appear relative to the wavelength of the excitation laser. When using a visible excitation laser, the Raman lines always appear in a range, where the absorption of water is negligibly small. Since the Raman signal intensity arising from water is small too, Raman spectroscopy works well in aqueous environments. This turns out to be a key advantage with regard to biological samples.

In the field of medical diagnostics, imaging Raman spectroscopy is a promising technique for identifying cancerous parts of tissue. A Raman microscope for the examination of skin in-vivo at the patient is already commercially available. However, like common Raman microscopes, the Raman microscopes known in the art can only detect Raman scattering arising from a single spot with a diameter in the range of micrometers. By contrast, potentially cancerous skin areas are in the range of at least some square millimeters. Standard point-by-point scanning is time-consuming. Even for an image being composed of some 100 pixels, the measurement time easily takes one hour. For examination at a patient or for fast-changing samples, this time span is far too long. To accelerate the measuring procedure, various techniques for a parallel data collection are described and known in the art. Of particular interest are full-throughput snapshot techniques, also called multichannel or integral field spectroscopy (IFS). They work without any tuning procedures and fundamentally sacrifice no light during the procedure.

EP 1 821 097 A1 describes the use of high wavenumber Raman spectroscopy for measuring tissue. The invention is related to an instrument for measuring a Raman signal of tissue, the instrument comprising a laser, a signal detection unit for measuring the Raman signal, and a fiber optic probe, wherein the fiber optic probe comprises one or more fibers for directing laser light onto the tissue and for collecting light that is scattered by the tissue and guiding the collected light away from the tissue towards the signal detection unit.

WO 2004/082474 A1, WO 2010/114361 A1, and WO 2010/114375 A1 for example describe further methods and instruments for measuring tissues, especially skin, for diagnostic purposes.

ANTHONY TSIKOURAS ET AL: "High-speed multifocal array scanning using refractive window tilting", BIOMEDICAL OPTICS EXPRESS, vol. 6, no. 10, 2 September 2015, pages 3737-3747, describes a device for fluorescence lifetime imaging microscopy (FLIM) comprising a light source, a first microlens array, scanning window galvanometers, a microscope, a second microlens array to collect and focus emitted light onto a two-dimensional 10x10 matrix of fibers of a fiber bundle, which are rearranged to a 1x100 line of fibers coupled to the input slit of a streak camera.

It is an object of the invention to solve the problem of time-consuming stepwise scanning of samples for imaging spectroscopy, in particular imaging Raman spectroscopy.

It is another object of the invention to solve the problem of providing more and/or alternative information about the sample, in a more accurate and quantitative manner.

These problems are solved according to the present invention by providing a device with the features of independent claim 1. Further advantageous features and embodiments of the present invention are laid out in the dependent claims.

Provided is a device for imaging spectroscopy for simultaneous spectrally and spatially resolved acquisition of all pixels of large-area samples, the device comprising:
a light source,
a signal detection unit for measuring the spectroscopic signal,
an optical system for directing light in form of a first light path onto the sample and for collecting light that is scattered, emitted or reflected by the sample and guiding the collected light away from the sample towards the signal detection unit in form of a second light path, wherein the optical system
   comprises:
   a fiber bundle provided for guiding the scattered, emitted or reflected light to the detection unit, wherein the fibers of the fiber bundle are arranged as
      two-dimensional fiber matrix at the sample side, and arranged as one row at the side of the signal detection unit,
   a first microlens array arranged in the first light path, whereby the first microlens array is arranged to focus the light onto the sample as well as to collect the light that is scattered, emitted or reflected by the sample,
   a second microlens array arranged in the second light path, whereby the second microlens array is arranged to focus the collected light individually into single fibers of the two-dimensional fiber matrix, and
   a square core fiber, which is connected to the light source, provided for guiding the light to the first light path.

Preferred according to the invention is a device, wherein the light source is a white light source, a light emitting diode, a white light laser, or a laser, selected from the group consisting of semiconductor lasers, solid state lasers, fiber lasers, dye lasers and gas laser. Especially preferred is that the light source has an excitation wavelength in the range of 250 nm to 1600 nm.

Preferred is also a device, wherein the signal detection unit is selected from Raman spectrographs, fluorescence imaging spectrographs, fluorescence lifetime imaging spectrographs, UV-Vis spectrographs, IR spectrographs, Michelson interferometers, Echelle spectrographs.

Especially preferred is that the square core fiber is connectable with a mechanic oscillator. Preferred according to the invention is also a device in that the bundle of fibers comprises at least 100 multimode fibers. Especially preferred is that the number of multimode fibers is at least 400.

Preferred is also a device, wherein the first and second microlens array comprise the identical number of microlenses.

Especially preferred is a device, wherein the number of micro lenses is identical to the number of multimode fibers.

Another object of the present invention is the use of the device according to present invention for imaging Raman spectroscopy of large-area samples.

A further object of the present invention is the use of the device according to present invention for imaging fluorescence lifetime spectroscopy of large-area samples.

A further object of the present invention is the use of the device according to present invention for imaging fluorescence spectroscopy of large-area samples.

A further object of the present invention is the use of the device according to present invention for imaging reflectance spectroscopy of large-area samples.

The basic idea of the present invention is to provide a device for simultaneous spectrally and spatially resolved acquisition of all pixels of large area samples without the need of scanning large area samples and to acquire the information from the pixels with separate measurements. A device is provided that uses a basic set up as known in the art, characterized in that two microlens arrays are arranged in the light path that is used for spectroscopy. The first microlens array is arranged in front of the large area sample in order to direct the light to the pixel that has to be investigated. The light that is directed to the sample in the first light path is therefore focused to distinct pixels.

The light that is scattered, emitted or reflected by the sample is then directed to the detection unit of the device according to the invention. As second microlens array is arranged in front of the fiber bundle that directs the light, scattered emitted or reflected by the sample, to the detection unit. Each lens of the microlens array is optically connected to one fiber of the fiber bundle. This means that each pixel on the sample is connected to one fiber of the fiber bundle. In order to receive the optic information from the pixel of the sample and to image this information, the single fibers of the fiber bundle are arranged in the row. A large-scale detector chip that is part of the detector unit is now in the position to image all spectral and spatial information from each pixel of the sample simultaneously.

The term "signal detector unit" as used with in the description of the present invention relates to any device that is in the position to collect spectral information that result from scattering, emitting or reflecting light from a sample under investigation. The detector unit is also in the position to collect spectral information from a great range of wavelengths that are used in imaging spectroscopy according to the invention. Suitable detector units that can be used in the context of the present invention must comprise features, properties and/or components that allow to fulfil the requirements according to the invention in respect to imaging spectroscopy. Such detector units may comprise wide field optics which compensate aberration. Detector units suitable for the present invention should also comprise large-scale detector chips like CCD or CMOS. Furthermore, the detector unit must be capable to produce images so that single light trails can be measured separately. Depending from the specific spectroscopic method the detector unit has to be adapted respectively. In some cases it is essential that the detector is gateable or phase sensitive. The detector unit must also comprise dispersive elements or compounds that are in the position to disperse the light. Suitable dispersive elements or compounds are for example prisms, volume holographic gratings, volume phase holographic gratings, reflection gratings, or combinations of gratings and prisms (so-called grisms). Useful may also be a cross disperse Echelle grating. A person skilled in the art is in the position to choose or arrange a setup for a detector unit that can be used according to the invention. It has to be pointed out that it is essential that large-scale detector chips have to be used in most cases. "Large-scale" in the context of the present invention means that the number of pixels is much higher than 1064 x 64.

Suitable detector units that can be used according to the present invention are commonly named as "spectrograph" or "spectrometer". As given in the definition above it is essential that the detector unit that can be used according to the invention must be in the position to detect a larger amount of spectra of different wavelengths at the same time. In the art those devices are named spectrograph. But this definition is not consistent in the art so that the suitable devices may be called in the art also spectrometer. As used with in the present invention the terms spectrograph and spectrometer are used as equivalents.

The present invention is explained in detail by the attached figures.
Fig. 1 shows the principle setup of a first embodiment of the device according to the invention.
Fig. 2 shows the principle setup of a second embodiment of the device according to the invention.
Fig. 3 shows the principle setup of a third embodiment of the device according to the invention.
Fig. 4 shows the function principle of fiber bundle-coupled IFS used for detecting spectra according to the invention.
Fig. 5 shows part of the letter "E" printed on a 10 Euro bill using the device according to the first embodiment of the invention.

The present invention is now described and explained in more detail. It shall be understood that the herein described embodiments are only for illustrative purposes. It is not intended to restrict the scope of the present invention to the preferred embodiments described herein. A person skilled in the art will be able to modify the described embodiments by not deviating from the scope of the present invention, which is defined by the appended claims.

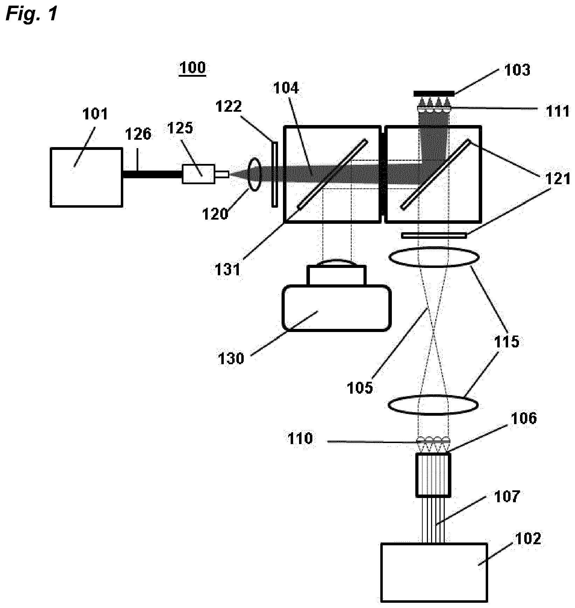

Referring to Figures 1 to 4 preferred embodiments for a setup of a device according to the invention are described.

Fig. 1 shows the principle setup of a first embodiment of the device according to the invention. Device 100 as shown in Fig.1 is a setup that is useful for Raman spectroscopy. In this case the light source is usually a laser light source. A light source 101 is provided and connected with a square core fiber 126 that is terminated with a fiber optic connector 125. First light path 104 is directed to a first long pass filter 121 which directs the light to the sample 103. Microlens array 111 is arranged in light path 104 and focuses the light onto pixels of sample 103. CCD camera 130 is provided for adjusting the light path 104 and to test the setup. Further optical components like collimator 120 and clean-up filter 122 are provided also. Second light path 105 directs the scattered light to the signal detection unit 102. Achromatic doublet 115 guides the image of the sample onto the surface of the fiber matrix 106. Second microlens array 110 is arranged in second light path 105 in order to direct the scattered light into the single fibers of fiber matrix 106. Fiber matrix 106 with outgoing fiber bundle 107 is arranged in order to guide the scattered light into signal detection unit 102.

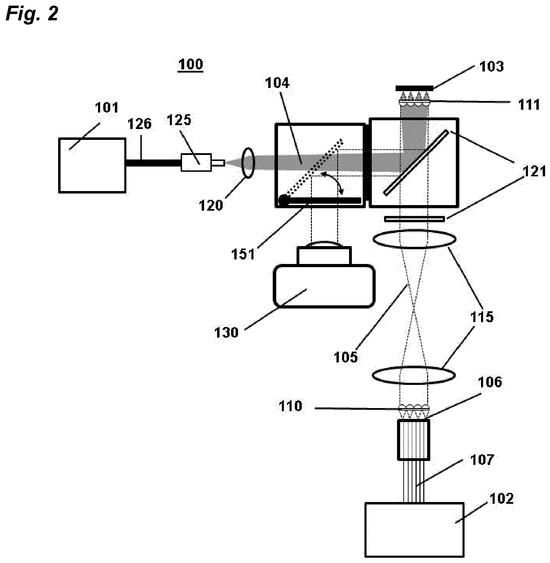

Fig. 2 shows the principle setup of a second embodiment of the device according to the invention. Device 100 shown in Fig. 2 is a setup that is useful for measuring fluorescence imaging and fluorescence life time imaging. In this case the light source is a visible light laser in order to excite fluorescence. A light source 101 is provided and first light path 104 is directed to sample 103 as described in Fig. 1. The other components are similar to the setup described in Fig. 1. CCD camera 130 is arranged to control the position of the sample. In the present set up a flipping mirror 151 is used in that context. According to the light type that is emitted by light source 101 some optical components may be omitted for the respective purpose. Microlens arrays 110, 111 are arranged in the first light path 104 and in the second light path 105 as described in Fig. 1.

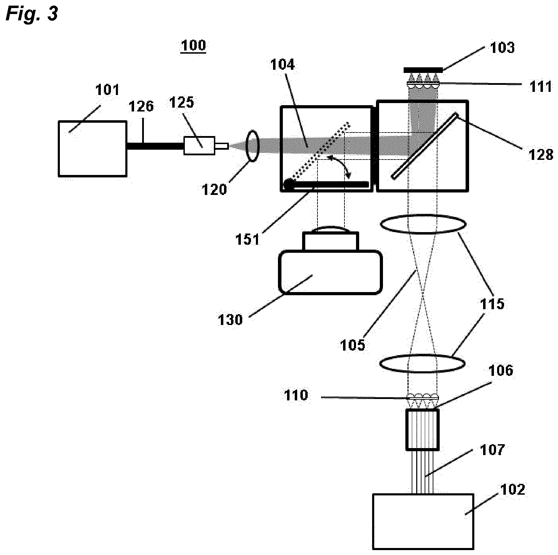

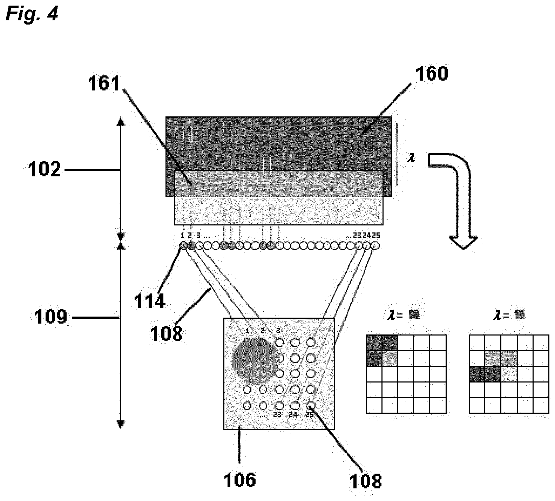

Fig. 3 shows the principle setup of a third embodiment of the device according to the invention. Device 100 shown in Fig .3 is a setup that is useful for measuring surface reflections using a light source with normal white light or white light lasers. Again, first light path 104 is directed to sample 103 and the reflected light is guided by a second light path 105 to the signal detection unit 102. In this set up a 50 : 50 mirror 128 is used in order to direct first light path 104 to sample 103. CCD camera 130 is provided with the same means as described in Fig. 2. Microlens arrays 110, 111 are arranged in the first light path 104 and in the second light path 105 as described in Fig. 1. Fig. 4 shows the setup of fiber bundle-coupled IFS used for detecting spectra according to the invention. The basic setup consists of two sections. The first section is fiber setup 109, the second section is signal detection unit 102.

Signal detection unit 102 consists of a CCD chip 160 that is used for imaging spectra. In order to enhance the capabilities of CCD chip 160 a volume phase holographic grating 161 is also provided. CCD chip 160 is capable for imaging a large amount of spectra simultaneously. The individual spectra are directed to the surface of CCD chip 160 by single fibers 108 that are arranged in form of the fiber bundle row 114.

Fiber setup 109 consists of the fiber matrix 106. For reasons of clarity and comprehensibility, only 25 fibers 108 are illustrated. Actually, the fiber matrix contains 400 fibers. Fiber matrix 106 has a square layout with a constant distance of single fibers 108. This setup can be used as signal detection unit 102 for the optical setups as described in Figs. 1 to 3.

Fig. 4 shows also the function principle of fiber bundle-coupled IFS. The image of the sample (black and grey semi circles) is focused on the fiber matrix of the image acquisition head. At the spectrograph side, the fibers are arranged in a one-dimensional row. After passing a spectral disperser (e.g. a volume phase holographic grating, VPHG), every fiber produces an individual intensity- and spectral-dependent light trace at the CCD chip. The raw signal of the CCD is evaluated by software and converted into a data cube that contains the entire spectral and spatial information.

The basic idea of IFS is slicing a two-dimensional image into single stripes and assembling them longitudinally in front of the entrance slit of a spectrograph. This can be performed by an image slicer, i.e. a mirror stack followed by a recombining optic, or by use of a fiber bundle. Fiber bundle-coupled IFS is also called FAST (fiber array spectral translation) or FRIS (fiber-reformatting imaging spectroscopy). At the sample side, the image of the investigated object, which may be a Raman, a fluorescence or a reflectance image, is focused at the surface of a two-dimensional fiber array. The fibers collect the signal of the image. At the spectrograph side, the fibers of the bundle are arranged in one row forming a pseudo-slit for the spectrograph. The signals emerging from each fiber are collimated, pass a dispersive element, and finally form spectral-dependent light traces on a CCD chip. A software evaluates the CCD raw signal and finally produces a data cube that contains the entire spectral and spatial information. In the end, the setup works as if every single fiber was connected to an individual single-channel spectrograph.

The setup described herein allows the recording of spatially-resolved large-scale (100 mm²) Raman, fluorescence, fluorescence lifetime, and reflectance images without scanning procedure. In the case of Raman imaging, the overall measurement time to receive the full spectral and spatial information was between 120 s and 2 s, according to the sample investigated, which corresponds to 300 and 5 ms, respectively, per pixel. Since the entire image is captured within one single exposure no additional time for moving the sample is required. The Raman images were generated by simply relating intensities at certain wavelength of the Raman spectra to pseudo colors. Even with this ordinary analysis method, high-contrast Raman images are gained that correspond to the structures shown by the camera. The results show great promise for being able to identify cancerous tissue in a next step.

The following examples and embodiments explain the invention in more details. It is not intended that the examples or herein described embodiments restrict the scope of the invention.

### Example 1

### Measuring device for Raman spectroscopy

As a light source, a fiber-coupled dual laser light source (Newport, USA, LS-2-7878-FC) that contains two switchable diode lasers with 784.5 and 785.5 nm was used. The maximum output power of each laser is 500 mW. The laser power was aligned to 230 mW measured in the sample plain.

The used spectrograph unit is a high performance fully refractive optical system that covers a spectral range from 465 to 930 nm with about 0.25 nm resolution. It employs a volume phase holographic grating (VPHG) and a liquid-nitrogen cooled 4096 × 4112 pixel CCD detector system. The VPGH, consisting of a phase-modulating dichromatic gelatin layer that is sealed between two glass plates, shows up to 80 % diffraction efficiency and minimally scattered light over the whole spectral range. *p3d,* an evaluation and data reduction software that was initially developed for astronomical purposes, has been used to calculate the spectra from the raw CCD image and to correlate them with the source position at the two-dimensional fiber array. Finally, a data cube is generated, which contains all spectral and spatial information for further data processing. The fiber-bundle contains 400 step index multimode fibers (110 µm core), forming a 20 × 20 matrix at the image acquisition side. The pitch between the fiber core centers is 0.5 mm, which is an acceptable tradeoff between resolution and crosstalk with regard to the scattering properties of skin samples.

The use of fiber bundle coupled IFS for imaging Raman spectroscopy is known in the art. However, previous setups were designed to examine samples with microscopic dimension, e.g. cells or tiny crystal fragments. The used setup according to the invention is capable to acquire Raman images from samples with a size up to 1 cm², which is the typical size of potentially cancerous skin parties. For comparison purposes two optic setups were developed. Acquisition optics 1 (AO1) allows recording of Raman images from square surfaces with 3.6 mm edge lengths, Acquisition optics 2 (AO2) is even suitable for a square area with 1 cm edge length. In order to receive large-area Raman images within one single exposure and without scanning, it is favorable to illuminate the whole area of interest as uniformly as possible with the excitation laser. This was performed by feeding the laser light with a square core fiber (F & T Fibers and Technology GmbH, Germany, VIS-IR 600 × 600 µm core). Highly multimode square core fibers, which have been designed initially for laser material processing, shape the incoming Gaussian-like beam to a top-hat intensity.

The setup of AO1 is as follows. The excitation laser light (784.5 nm) is fed via the square core glass fiber, passes a collimator and a 785 nm clean-up filter and is finally guided to the sample by use of a 45° dichroic mirror that is transparent for Stokes-shifted Raman signals. An achromatic doublet focuses the laser light on the sample, resulting in a square laser spot with 4.1 mm edge length, and collects the arising fluorescence or Raman scattering. in the case of a uniformly illuminated square, the power density of 14 mW / mm². It is worth to note that single channel Raman microscopes known in the art work with far higher power densities. A further dichroic mirror takes visible light arising from the sample and guides it to a camera that is used to position the sample. A 785 nm long pass filter blocks the excitation laser light and Rayleigh scattering. A second achromatic doublet (*f* = 125 mm, respectively) focuses the image of the sample, e.g. the arising Raman signals, onto the surface of the fiber matrix.

A weak point in the design of AO1 is the poor coupling efficiency at the side of the fiber matrix. The fiber matrix has an area of 100 mm², however only 4 mm² consist of fiber cores. To improve the situation, a further optic unit (AO2) with a first microlens array (MLA) in front of the fiber matrix was used according to the invention and is shown in Fig. 1. The first MLA is intended to focus the signal into the fiber cores and therewith reduce losses at blind areas of the fiber matrix. A second MLA was used to increase the image field at the sample side to 10 × 10 mm, which was a target for future skin cancer detection. Focusing the excitation laser light with 400 small spots on the sample should allow an increasing of the Raman signal at the points of interest. A pair of relay lenses (doublets *f* = 50 mm) guides the image from the second to the first MLA. This setup has been used for test measurements.

### Exemplary measuring result: 10 Euro bank bill

A 10 Euro bank bill is put on 1 mm thick calcium fluoride slide and placed on a XY-stage over AO1 or AO2, respectively.

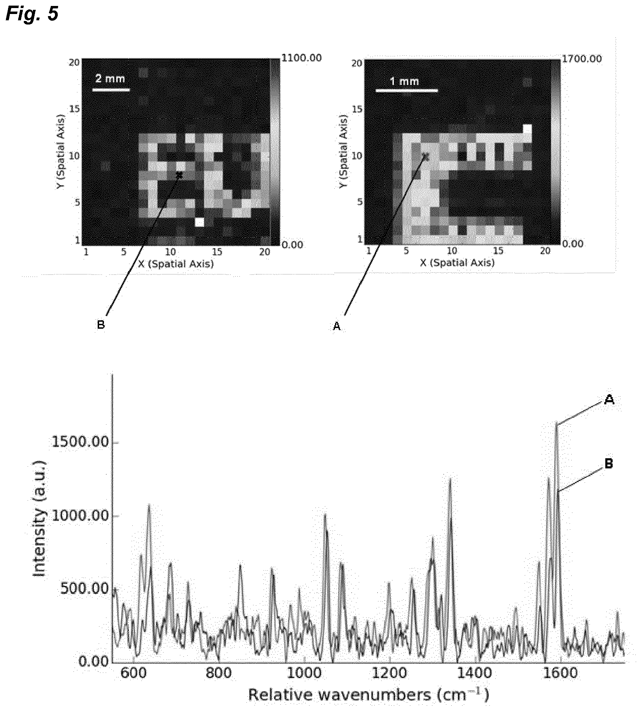

Fig. 5 shows a comparison of AO1 and the MLA-based AO2 by recording Raman images of the lettering "EURO" printed on a 10 Euro bill. Upper left: Pseudo color Raman image of a part of the "E" achieved with AO1. Upper right: Pseudo color Raman image of the letters "EU" achieved with AO2. Both Raman images base on the intensity at 1567 cm⁻¹, which corresponds to the distribution of the printing ink. Below: Comparison of Raman spectra received with AO1 and AO2 at the positions **A** and **B,** respectively (10 s recording time per laser).

The measurement was performed with AO1 and with microlens array-based AO2. The part of the bill with the lettering "EURO" was placed directly over the sample-side MLA. The distance to the MLA surface was about 0.6 mm, which is approximately the focus length. The measurement was performed with only one laser (784.5 cm⁻¹). The fluorescence was removed by the rolling circle method. For comparison the Raman spectra of the "E"-part received with AO1 at 784.5 cm⁻¹ were likewise background-removed with the rolling circle method. Measurement time was 10 s in both cases. Fig. 5 shows the results. The pseudo color Raman images show the Letter "EU" recorded with AO2 and the part of the "E" recorded with AO1 according to intensity of the Raman signal at 1576 cm⁻¹. The white square at (13/3) is due to a broken fiber of the bundle. In the right Raman image it appears at (18/13) since the bill was accidently turned by 90 degrees. The intensity of the spectra received from the printing ink of the "E" is similar, regardless if they were recorded from a 13 mm² surface with MO1 or from the 1 cm² surface with AO2. Obviously the MLAs compensate the enlargement of the image field very well.

### Example 2

### Measuring device for fluorescence imaging and fluorescence lifetime imaging (FLIM)

The microlens array-based (MLA-based) setup as described in Example 1 is also suitable for fluorescence imaging und fluorescence lifetime imaging (FLIM). Usually, fluorescence is excited with visible light. Fig. 2 shows a setup for excitation with a violet 405 nm laser light. A 45° long pass dichroic mirror reflects the excitation light into the direction of the sample. The fluorescence arising from the sample is separated from the excitation light by use of a 405 nm long pass filter and guided to the fiber matrix. The MLAs again focus the excitation light at the sample and into the front surfaces of the fibers at the fiber array, respectively. The detection unit determines the fluorescence spatially and spectrally resolved. Software evaluates the CCD raw signal. Finally, a data cube that contains the full spectral and spatial information is provided, from which fluorescence images can be reconstructed.

Even FLIM can be performed with this setup. For FLIM, the laser light needs to be appropriately modulated. Furthermore, a detector unit that allows time resolved or phase sensitive measurements is needed. A straight forward approach is to use a detector chip, e.g. an ICCD chip, which can be time gated in combination with a pulsed laser. The detector chip is activated only for a certain time interval and with a certain delay after the laser pulse. By shifting the delay, the fluorescence decay curves can be recorded step-by-step. Another embodiment is to measure the fluorescence decay time indirectly via the phase shift. When a fluorescent sample is excited with sinusoidal intensity modulated light, the emitted fluorescence light is intensity modulated as well, but slightly phase shifted, because fluorescence occurs time delayed. To translate a nanosecond fluorescence decay time into a phase shift of about 40°, the laser has to be modulated in the range of about 20 MHz.
Comparatively recently developed solid state detector chips with so-called in-pixel demodulation allow a phase-sensitive detection of the light signal. From the measured phase shift of the fluorescence signal with reference to the excitation light, decay times can be calculated. Both FLIM embodiments finally provide a four-dimensional data array that contains besides the spectral and spatial information also the decay times.

### List of reference signs

- 100: Device for imaging spectroscopy
- 101: light source, e.g. laser
- 102: signal detection unit
- 103: sample
- 104: first light path
- 105: second light path
- 106: fiber matrix
- 107: fiber bundle
- 108: fiber
- 109: fiber setup
- 110: microlens array
- 111: microlens array
- 114: fiber bundle row
- 115: doublet
- 120: collimator
- 121: long pass filter (0° or 45°, respectively)
- 122: clean-up filter
- 125: fiber optic connector
- 126: square core fiber
- 128: 50:50 mirror
- 130: CCD camera
- 131: long pass filter
- 151: flipping mirror
- 160: CCD chip
- 161: volume phase holographic grating (VPHG)

## Claims

1. Device (100) for imaging spectroscopy for simultaneous spectrally and spatially resolved acquisition of all pixels of large-area samples (103), the device (100) comprising:
a light source (101),
a signal detection unit (102) for measuring the spectroscopic signal,
an optical system for directing light in form of a first light path (104) onto the sample (103) and for collecting light that is scattered, emitted or reflected by the sample (103) and guiding the collected light away from the sample towards the signal detection unit (102) in form of a second light path (105), wherein the optical system comprises:
a fiber bundle (107) provided for guiding the scattered, emitted or reflected light to the signal detection unit (102), wherein the fibers of the fiber bundle are arranged as two-dimensional fiber matrix (106) at the sample side, and
arranged as one row (114) at the side of the signal detection unit (102), a first microlens array (111) arranged in the first light path (104), whereby the first microlens array (111) is arranged to focus the light onto the sample (103) as well as to collect the light that is scattered,
emitted or reflected by the sample (103), a second microlens array (110) arranged in the second light path (105), whereby the second microlens array (110) is arranged to focus the collected light individually into single fibers (107, 108) of the two-dimensional fiber matrix (106),
and a square core fiber (126), which is connected to the light source (101), provided for guiding the light to the first light path (104).

2. Device (100), according to claim 1, wherein the light source (101) is a white light source, a light emitting diode, a white light laser, or a laser, selected from the group consisting of semiconductor lasers, solid state lasers, fiber lasers, dye lasers and gas laser.

3. Device (100), according to claim 2, wherein the light source (101) has an excitation wavelength in the range of 250 nm to 1600 nm.

4. Device (100), according to claim 1, wherein the signal detection unit (102) is selected from Raman spectrographs, fluorescence imaging spectrographs, fluorescence lifetime imaging spectrographs, UV-Vis spectrographs, IR spectrographs, Michelson interferometers, Echelle spectrographs.

5. Device (100), according to claim 1, wherein the square core fiber is connectable with a mechanic oscillator.

6. Device (100), according to anyone of the preceding claims, wherein the bundle of fibers (106, 107) comprises at least 100 multimode fibers (108).

7. Device (100), according to claim 6, wherein the number of multimode fibers (108) is at least 400.

8. Device (100), according to anyone of the preceding claims, wherein the first (111) and second microlens array (110) comprise the identical number of microlenses.

9. Device (100), according to claim 8, wherein the fibers are multimodal fibers (108) and wherein the number of micro lenses is identical to the number of said multimode fibers (108).

10. Use of the device according to at least one of said preceding claims for imaging Raman spectroscopy of large-area samples.

11. Use of the device according to at least one of the claims 1 to 9 for imaging fluorescence lifetime spectroscopy of large-area samples.

12. Use of the device according to at least one of the claims 1 to 9 for imaging fluorescence spectroscopy of large-area samples.

13. Use of the device according to at least one of the claims 1 to 9 for imaging reflectance spectroscopy of large-area samples.

## Patentansprüche

1. Vorrichtung (100) für die bildgebende Spektroskopie für die gleichzeitige spektral und räumlich aufgelöste Erfassung aller Pixel von großflächigen Proben (103), die Vorrichtung (100) umfassend:
eine Lichtquelle (101), eine Signaldetektionseinheit (102) zur Messung des spektroskopischen Signals, ein optisches System, um Licht in Form eines ersten Strahlengangs (104) auf die Probe (103) zu richten, und
zum Sammeln von Licht, das durch die Probe (103) gestreut, emittiert oder reflektiert wird, und um das gesammelte Licht weg von der Probe in Richtung der Signaldetektionseinheit (102) in Form eines zweiten Strahlengangs (105) zu lenken, worin das optische System umfasst:
ein Faserbündel (107), vorgesehen, um das gestreute, emittierte oder reflektierte Licht zur Signaldetektionseinheit (102) zu lenken, worin die Fasern des Faserbündels als zweidimensionale Fasermatrix (106) auf der Probenseite angeordnet sind, und als eine Reihe (114) auf der Seite der Signaldetektionseinheit (102) angeordnet sind,
ein erstes Mikrolinsenarray (111), angeordnet in dem ersten Strahlengang (104), wobei das erste Mikrolinsenarray (111) angeordnet ist, um das Licht auf die Probe (103) zu fokussieren, wie auch das Licht, das von der Probe (103) gestreut, emittiert oder reflektiert wird, zu sammeln,
ein zweites Mikrolinsenarray (110), angeordnet in dem zweiten Strahlengang (105), wobei das zweite Mikrolinsenarray (110) angeordnet ist, um das gesammelte Licht individuell in einzelne Fasern (107, 108) der zweidimensionalen Fasermatrix (106) zu fokussieren,
und eine Faser mit quadratischem Kern (126), welche mit der Lichtquelle (101) verbunden ist, vorgesehen, um das Licht zu dem ersten Strahlengang (104) zu leiten.

2. Vorrichtung (100), gemäß Anspruch 1, worin die Lichtquelle (101) eine Weißlichtquelle, eine Licht emittierende Diode, ein Weißlichtlaser oder ein Laser, ausgewählt aus der Gruppe, bestehend aus Halbleiterlasern, Festkörperlasern, Faserlasern, Farbstofflasern und Gaslasern.

3. Vorrichtung (100), gemäß Anspruch 2, worin die Lichtquelle (101) eine Anregungswellenlänge im Bereich von 250 nm bis 1600 nm hat.

4. Vorrichtung (100), gemäß Anspruch 1, worin die Signaldetektionseinheit (102) ausgewählt ist aus Raman-Spektrographen, bildgebenden Fluoreszenz-Spektrographen, bildgebenden Fluoreszenzlebensdauer-Spektrographen, UV-Vis-Spektrographen, IR-Spektrographen, Michelson-Interferometern, Echelle-Spektrographen.

5. Vorrichtung (100), gemäß Anspruch 1, worin die Faser mit quadratischem Kern mit einem mechanischen Oszillator verbindbar ist.

6. Vorrichtung (100), gemäß irgendeinem der vorangehenden Ansprüche, worin das Faserbündel (106, 107) wenigstens 100 Multimodefasern (108) umfasst.

7. Vorrichtung (100), gemäß Anspruch 6, worin die Anzahl der Multimodefasern (108) wenigstens 400 ist.

8. Vorrichtung (100), gemäß irgendeinem der vorangehenden Ansprüche, worin das erste (111) und zweite Mikrolinsenarray (110) die identische Anzahl von Mikrolinsen umfassen.

9. Vorrichtung (100), gemäß Anspruch 8, worin die Fasern multimodale Fasern (108) sind, und worin die Anzahl an Mikrolinsen identisch zur Anzahl der genannten Multimodefasern (108) ist.

10. Verwendung der Vorrichtung gemäß wenigstens einem der genannten vorangehenden Ansprüche zur bildgebenden Raman-Spektroskopie von großflächigen Proben.

11. Verwendung der Vorrichtung gemäß wenigstens einem der Ansprüche 1 bis 9 zur bildgebenden Fluoreszenzlebensdauer-Spektroskopie von großflächigen Proben.

12. Verwendung der Vorrichtung gemäß wenigstens einem der Ansprüche 1 bis 9 zur bildgebenden Fluoreszenz-Spektroskopie von großflächigen Proben.

13. Verwendung der Vorrichtung gemäß wenigstens einem der Ansprüche 1 bis 9 zur bildgebenden Reflexionsspektroskopie von großflächigen Proben.

## Revendications

1. Dispositif (100) d'imagerie par spectroscopie permettant l'acquisition à résolutions spectrale et spatiale simultanées de tous les pixels d'échantillons de grande surface (103), le dispositif (100) comprenant :
une source de lumière (101),
une unité de détection de signal (102) permettant de mesurer le signal spectroscopique,
un système optique permettant de diriger la lumière sous la forme d'un premier trajet lumineux (104) sur l'échantillon (103), de collecter la lumière dispersée, émise ou réfléchie par l'échantillon (103) et de guider la lumière collectée en l'éloignant de l'échantillon vers l'unité de détection de signal (102) sous la forme d'un second trajet lumineux (105), le système optique comprenant :
un faisceau de fibres (107) prévu pour guider la lumière dispersée, émise ou réfléchie vers l'unité de détection de signal (102), les fibres du faisceau de fibres étant agencées sous forme de matrice de fibres bidimensionnelle (106) du côté de l'échantillon, et agencées sous forme de rangée unique (114) du côté de l'unité de détection de signal (102),
un premier réseau de microlentilles (111) agencé dans le premier trajet lumineux (104), le premier réseau de microlentilles (111) étant agencé de manière à focaliser la lumière sur l'échantillon (103) et à collecter la lumière dispersée, émise ou réfléchie par l'échantillon (103),
un second réseau de microlentilles (110) agencé dans le second trajet lumineux (105), le second réseau de microlentilles (110) étant agencé de manière à focaliser la lumière collectée individuellement en fibres uniques (107, 108) de la matrice de fibres bidimensionnelle (106),
et une fibre à âme carrée (126), reliée à la source de lumière (101) et prévue pour guider la lumière vers le premier trajet lumineux (104).

2. Dispositif (100) selon la revendication 1, dans lequel la source de lumière (101) est une source de lumière blanche, une diode électroluminescente, un laser à lumière blanche ou un laser choisi dans le groupe constitué par les lasers à semi-conducteurs, les lasers à l'état solide, les lasers à fibre, les lasers à colorant et les lasers à gaz.

3. Dispositif (100) selon la revendication 2, dans lequel la source de lumière (101) présente une longueur d'onde d'excitation située dans la plage allant de 250 nm à 1 600 nm.

4. Dispositif (100) selon la revendication 1, dans lequel l'unité de détection de signal (102) est choisie parmi les spectrographes Raman, les spectrographes d'imagerie par fluorescence, les spectrographes d'imagerie de durée de vie en fluorescence, les spectrographes à UV-visible, les spectrographes à infrarouge, les interféromètres de Michelson et les spectrographes d'Echelle.

5. Dispositif (100) selon la revendication 1, dans lequel la fibre à âme carrée peut être reliée à un oscillateur mécanique.

6. Dispositif (100) selon l'une quelconque des revendications précédentes, dans lequel le faisceau de fibres (106, 107) comprend au moins 100 fibres multimode (108).

7. Dispositif (100) selon la revendication 6, dans lequel le nombre de fibres multimode (108) est d'au moins 400.

8. Dispositif (100) selon l'une quelconque des revendications précédentes, dans lequel les premier (111) et second (110) réseaux de microlentilles comprennent un nombre identique de microlentilles.

9. Dispositif (100) selon la revendication 8, dans lequel les fibres sont des fibres multimode (108) et dans lequel le nombre de microlentilles est identique au nombre desdites fibres multimode (108).

10. Utilisation du dispositif selon au moins l'une des revendications précédentes pour l'imagerie par spectroscopie Raman d'échantillons de grande surface.

11. Utilisation du dispositif selon au moins l'une des revendications 1 à 9 pour l'imagerie par spectroscopie de durée de vie en fluorescence d'échantillons de grande surface.

12. Utilisation du dispositif selon au moins l'une des revendications 1 à 9 pour l'imagerie par spectroscopie de fluorescence d'échantillons de grande surface.

13. Utilisation du dispositif selon au moins l'une des revendications 1 à 9 pour l'imagerie par spectroscopie de réflectance d'échantillons de grande surface.
